# EUROPEAN PATENT APPLICATION

(11) **EP 4 691 452 A1**
(43) Date of publication of application: **11.02.2026**
(21) Application number: 24779469.6
(22) Date of filing: 13.03.2024
(51) Int. Cl.: A61K 8/37, A61K 8/06, A61K 8/27, A61K 8/92, A61Q 17/04, A61Q 19/00

(54) **COSMETIC COMPOSITION**

(30) Priority: 27.03.2023 JP 2023050332
(71) Applicant: Shiseido Company, Ltd., Chuo-ku Tokyo 104-0061 (JP)
(72) Inventor: HIROISHI, Megumi, Tokyo 104-0061 (JP)
(74) Representative: Ter Meer Steinmeister & Partner
(86) International application number: PCT/JP2024/009796
(87) International publication number: WO 2024/203340

(57) **Abstract**

Provided is a cosmetic composition having an ultraviolet protection effect while capable of improving the wavelength conversion efficiency of an ultraviolet wavelength conversion substance.

A cosmetic composition, comprising (A) an ultraviolet wavelength conversion substance; (B) Compound I having a structure of formula (I) below; and (C) an oil.

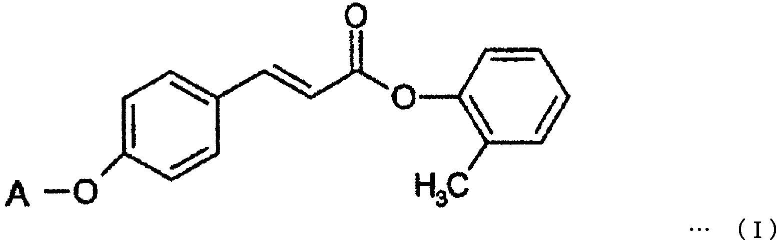

## Description

### FIELD

The present invention relates to a cosmetic composition.

### BACKGROUND

Ultraviolet rays are said to cause oxidation of sebum and damage to cellular DNA by generating free radicals *in vivo.* The adverse effects on the skin due to such effect of ultraviolet rays include negative effects such as skin cancer, photoaging, spots, wrinkles, and inflammation, and are not preferable from the viewpoints of health and beauty.

Examples of the types of ultraviolet rays include medium-wavelength ultraviolet rays (UVB region: wavelengths of 290 to 320 nm) that are known to cause sunburn and inflammation and long-wavelength ultraviolet rays (UVA region: wavelengths of 320 to 400 nm) that are known to cause photoaging.

Numerous measures for protecting the skin from ultraviolet rays have been developed to date.

For example, PTL 1 discloses a composition, as a novel composition having a cell-activating action that utilizes ultraviolet rays, comprising (A) an ultraviolet wavelength conversion substance; (B) a dispersant; (C) an ultraviolet absorber and/or ultraviolet-scattering agent; and (D) an oil, wherein blending amount of the (B) dispersant is 1% by weight or greater.

PTL 2 discloses an ultraviolet absorber having an absorption capacity in both the UVA region and the UVB region and having an ultraviolet-suppressing effect in a wide range of wavelengths, wherein the ultraviolet absorption capacity in the UVA and UVB regions increases with the passage of time during ultraviolet irradiation. More specifically, the ultraviolet absorber of PTL 2 comprises, as an active component, a compound represented by general formula I: 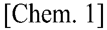 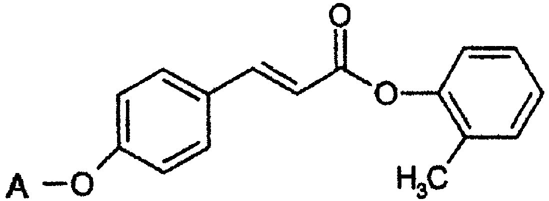 wherein -OA represents an alkoxy group.

### [CITATION LIST]

### [PATENT LITERATURE]

[PTL 1] WO 2020/204193
[PTL 2] WO 2009/041098

### SUMMARY

### [TECHNICAL PROBLEM]

To achieve both a cell-activating effect and an ultraviolet protection effect, for example, compositions in which an ultraviolet wavelength conversion substance and an ultraviolet absorber are blended have been considered, as in PTL 1 described above.

However, when an ultraviolet wavelength conversion substance is used in combination with an ultraviolet absorber, the wavelength conversion efficiency thereof may deteriorate. There is a problem that both an ultraviolet protection effect and a cell-activating effect cannot be achieved.

The present invention aims to improve the above circumstances, and an object thereof is to provide a cosmetic composition having an ultraviolet protection effect while capable of improving the wavelength conversion efficiency of an ultraviolet wavelength conversion substance.

### [SOLUTION TO PROBLEM]

The present invention that achieves the above object is as follows.

### <Aspect 1>

A cosmetic composition, comprising
(A) an ultraviolet wavelength conversion substance;
(B) Compound I having a structure of formula (I) below: 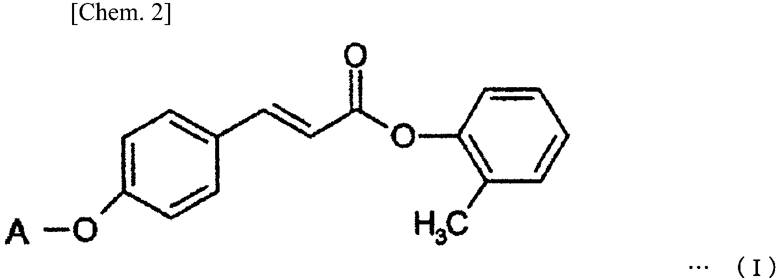 wherein -OA represents an alkoxy group; and
(C) an oil.

### <Aspect 2>

The composition according to Aspect 1, wherein the component (A) is a zinc oxide phosphor.

### <Aspect 3>

The composition according to Aspect 1 or 2, wherein a content of the component (B) is 0.5 to 20% by mass.

### <Aspect 4>

The composition according to any one of Aspects 1 to 3, wherein the component (B) is 2-methylphenyl 4-methoxycinnamate.

### <Aspect 5>

The composition according to any one of Aspects 1 to 4, further comprising (D) a UVA absorber.

### <Aspect 6>

The composition according to any one of Aspects 1 to 5, wherein the component (C) comprises a polar oil.

### <Aspect 7>

The composition according to any one of Aspects 1 to 6, which is a water-in-oil cosmetic composition.

### <Aspect 8>

The composition according to any one of Aspects 1 to 6, which is an oil-in-water cosmetic composition.

### [ADVANTAGEOUS EFFECTS OF INVENTION]

According to the present invention, a cosmetic composition having an ultraviolet protection effect while capable of improving the wavelength conversion efficiency of an ultraviolet wavelength conversion substance can be provided.

### DESCRIPTION OF EMBODIMENTS

Hereinafter, embodiments of the present invention will be described in detail. The present invention is not limited to the following embodiments, and various modifications can be made within the scope of the invention.

### <<Cosmetic composition>>

The cosmetic composition of the present invention (hereinafter, also simply referred to as "composition of the present invention") is
a cosmetic composition, comprising
(A) an ultraviolet wavelength conversion substance;
(B) Compound I having a structure of formula (I) below: 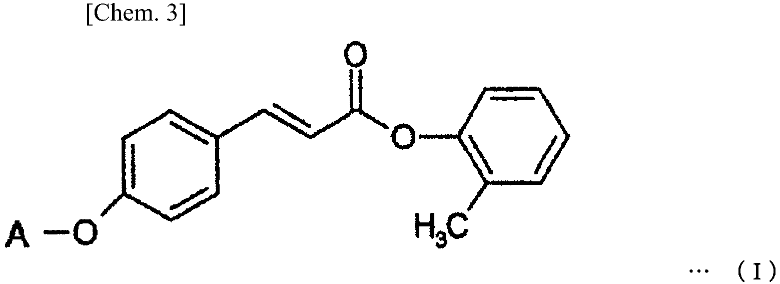 wherein -OA represents an alkoxy group; and
(C) an oil.

Through intensive research by the present inventors, it was discovered that by using Compound I having a structure of formula (I) described above (hereinafter, also simply referred to as "Compound I") and an ultraviolet wavelength conversion substance in combination, the wavelength conversion efficiency of the ultraviolet wavelength conversion substance is improved.

The excitation wavelength of a zinc oxide phosphor is near 365 nm (UVA wavelength region). Therefore, it is considered that when ultraviolet absorption intensity of a cosmetic composition near 365 nm is large, wavelength conversion efficiency of an ultraviolet wavelength conversion substance is reduced.

The inventors of the invention of the present case have surprisingly discovered that, compared to conventional cosmetic compositions containing ethylhexyl salicylate or ethylhexyl methoxycinnamate, which are common UVB absorbers, and an ultraviolet wavelength conversion substance, the composition of the present invention containing Compound I, which can be used as a UVA and UVB absorber, and an ultraviolet wavelength conversion substance significantly improves the wavelength conversion efficiency of the ultraviolet wavelength conversion substance.

### <Component (A): ultraviolet wavelength conversion substance>

The composition of the present invention comprises an ultraviolet wavelength conversion substance as the component (A). By including an ultraviolet wavelength conversion substance, a cell-activating effect can be obtained. By activating cells, damage to the skin when irradiated with ultraviolet rays can be alleviated, or condition of the skin can be proactively improved. Thus, the composition of the present invention is suitable for collagen production and hyaluronic acid production by fibroblasts and suppression of damage due to photoaging, and is suitable for suppressing oxidative stress in keratocytes, enhancing barrier function, suppressing inflammatory responses, and suppressing collagen glycation and angiogenesis in the skin. Regarding cell-activating effects, the description of PTL 1 can be appropriately referenced.

The ultraviolet wavelength conversion substance refers to a substance that converts the wavelength of ultraviolet rays contained in incident light and emits outgoing light at a wavelength longer than the wavelength of the ultraviolet rays. The ultraviolet rays applied to the ultraviolet wavelength conversion substance may include UVA (ultraviolet A rays, wavelength: 320 nm to 400 nm), UVB (ultraviolet B rays, wavelength: 280 nm to 320 nm), and UVC (ultraviolet C rays, wavelength: 100 nm to 280 nm). In some embodiments, the ultraviolet rays may consist of light having a peak wavelength of 200 nm to 400 nm, and may be ultraviolet rays from incident light, for example, sunlight, or artificially generated ultraviolet rays.

The outgoing light emitted by the ultraviolet wavelength conversion substance has a wavelength longer than those of ultraviolet rays, and may preferably have a peak wavelength of 500 nm to 700 nm. The outgoing light is not particularly limited and, for example, may have one or more peaks at 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm, 700 nm, or within any range specified by a combination of these numerical values. The outgoing light may be red light, orange light, green light, or blue light. In some embodiments, the ultraviolet wavelength conversion substance, when excited by excitation light of 200 nm to 400 nm, emits light exhibiting a dominant wavelength of 500 nm to 700 nm.

In the present invention, cumulative fluorescence value, for example, as in the Examples, can be measured by forming a coating of the composition on a surface of a base material and measuring the fluorescence intensity when irradiated with ultraviolet rays using a spectrofluorometer. As the base material, a resin substrate, such as that of polymethyl methacrylate (PMMA), nylon, or an acrylic plate; or an inorganic plate, such as that of glass or quartz, can be used. For example, a PMMA plate (also referred to as "S plate": refer to Japanese Registered Patent Publication No. 4453995) provided with V-shaped grooves on the surface can be used. Fluorescence intensity can be measured in the same manner as cumulative fluorescence value. The fluorescence intensity may be measured as a fluorescence value at a specific single wavelength, or as a cumulative value in a specific wavelength region.

In the present invention, as the ultraviolet wavelength conversion substance, an inorganic ultraviolet wavelength conversion substance, an organic ultraviolet wavelength conversion substance, or a mixture thereof may be used.

Inorganic ultraviolet wavelength conversion substance refers to an ultraviolet wavelength conversion substance that is an inorganic compound. Specific examples of the inorganic ultraviolet wavelength conversion substance are not particularly limited and, for example, include phosphors formed by doping an inorganic compound to impart fluorescence, for example, a blue phosphor comprising amorphous silica particles, cerium, and phosphorus and/or magnesium, described in Japanese Registered Patent Publication No. 6424656; a red phosphor comprising a compound in which a mixed crystal of an alkaline earth metal sulfide and a gallium compound is activated with europium, described in Japanese Registered Patent Publication No. 6361416; a phosphor of zinc oxide, described in WO 2018/004006; a phosphor of zinc oxide, described in Japanese Unexamined Patent Publication (Kokai) No. 2018-131422; and an inorganic phosphor, described in Japanese Unexamined Patent Publication (Kokai) No. 5-117127. More specifically, the inorganic phosphor is one or a plurality of phosphors selected from phosphors in which zinc oxide, which can be represented as ZnO:Zn, Zn_{1+z}, or ZnO₁₋ₓ, is doped with a sulfur-containing compound, such as a sulfide and/or sulfate, for example, zinc sulfide or zinc sulfate, described in WO 2018/004006; phosphors of magnesium titanate in which magnesium titanate, such as MgTiO₃ or Mg₂TiO₄, is doped with manganese (hereinafter, a phosphor derived from magnesium titanate will be referred to as "magnesium titanate phosphor"); and calcium phosphate phosphors in which calcium phosphate, such as Ca(H₂PO₄)₂, CaHPO₄, Ca₃(PO₄)₂, is doped with cerium. Among these, in particular, the composition of the present invention preferably comprises a phosphor derived from zinc oxide (i.e., "zinc oxide phosphor").

In the present invention, the inorganic ultraviolet wavelength conversion substance, which is an inorganic phosphor, may undergo a surface treatment. Examples of the surface treatment include silane compound treatment (such as octyltriethoxysilane), silicone compound treatment, fluorine-modified silicone compound treatment, fluorine compound treatment, higher fatty acid treatment (such as stearic acid), higher alcohol treatment, fatty acid ester treatment, metal soap treatment, amino acid treatment, and alkyl phosphate treatment.

Organic ultraviolet wavelength conversion substance refers to an ultraviolet wavelength conversion substance that is an organic compound. Specific examples thereof are not particularly limited and, for example, include phycobiliproteins such as phycocyanins (allophycocyanin, C-phycocyanin, and R-phycocyanin), phycoerythrocyanin, and phycoerythrins (B-phycoerythrin, b-phycoerythrin, C-phycoerythrin, and R-phycoerythrin); naturally derived and synthetic components such as vitamin A, β-carotene, vitamin K, vitamin B1, vitamin B2, vitamin B6, vitamin B12, folic acid, niacin, salicylic acid, lycopene, chili pepper extract, chili pepper colorant, paprika colorant, gardenia colorant, safflower colorant, turmeric colorant, cochineal colorant, perilla colorant, red cabbage colorant, flavonoids, carotenoids, quinoids, porphyrins, anthocyanins, and polyphenols; and colorants such as Red No. 401, Red No. 227, Red No. 504, Red No. 218, Orange No. 205 P, Yellow No. 4, Yellow No. 5, Green No. 201, pyranine concentrate, Blue No. 1, 2,4-diaminophenoxyethanol hydrochloride, Alizurin Purple SS, Purple No. 401, Black No. 401, Helindon Pink, Yellow No. 401, Benzidine Yellow G, Blue No. 404, Red No. 104, and meta-aminophenol.

The ultraviolet wavelength conversion substance may be obtained by a method such as extraction from natural products such as plants and algae, or by an artificial method such as chemical synthesis. For example, phycobiliprotein may be prepared by extracting algae such as blue-green algae, such as *Spirulina platensis,* or red algae, such as *Porphyridium purpureum,* by a method described in, for example, Japanese Registered Patent Publication No. 4048420, Japanese Registered Patent Publication No. 4677250, or Japanese Registered Patent Publication No. 3303942. The zinc oxide phosphor may be manufactured by a method described in, for example, WO 2018/004006, Japanese Unexamined Patent Publication (Kokai) No. 2018-131422, or Japanese Unexamined Patent Publication (Kokai) No. 5-117127. The magnesium titanate phosphor may be manufactured by a method described in Japanese Unexamined Patent Publication No. 2017-88719. The calcium phosphate phosphor may be manufactured by a method described in WO 2018/117117.

In the composition of the present invention, the content of the ultraviolet wavelength conversion substance is not particularly limited and, for example, may be 0.001% by mass or greater, 0.005% by mass or greater, 0.01% by mass or greater, 0.05% by mass or greater, 0.1% by mass or greater, 0.5% by mass or greater, 1.0% by mass or greater, 1.5% by mass or greater, 2.0% by mass or greater, 2.5% by mass or greater, 3.0% by mass or greater, 3.5% by mass or greater, 4.0% by mass or greater, 4.5% by mass or greater, or 5.0% by mass or greater, and may be 50% by mass or less, 45% by mass or less, 40% by mass or less, 35% by mass or less, 30% by mass or less, 25% by mass or less, 20% by mass or less, 15% by mass or less, 10% by mass or less, 8.0% by mass or less, or 6.0% by mass or less, relative to the entire composition.

### <Component (B): Compound I>

Compound I contained in the composition of the present invention has a structure of formula (I) below: 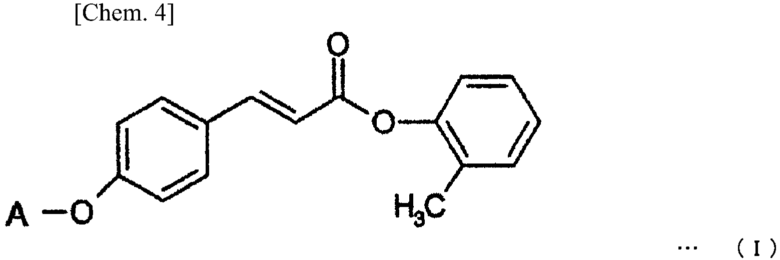 wherein -OA represents an alkoxy group.

In the formula (I), -OA represents an alkoxy group, and more specifically, includes, for example, a methoxy group and an ethoxy group, but is not limited thereto. This Compound I can be used as a UVA and UVB absorber.

In the present invention, Compound I may consist of the same structure as the compound represented by general formula I disclosed in PTL 2. In the present invention, Compound I as the component (B), in particular, is preferably 2-methylphenyl 4-methoxycinnamate.

In the composition of the present invention, from the viewpoint of further exhibiting the effect of the present invention, it is preferable that Compound I be at least in a partially dissolved state, particularly 50% by mass or greater in a dissolved state, more particularly 90% by mass or greater in a dissolved state, even more particularly 99% by mass or greater in a dissolved state, and most particularly 100% by mass in a dissolved state.

In the composition of the present invention, the content of Compound I is not particularly limited and, for example, may be 0.1% by mass or greater, 0.5% by mass or greater, 1.0% by mass or greater, 1.5% by mass or greater, 2.0% by mass or greater, 2.5% by mass or greater, 3.0% by mass or greater, 3.5% by mass or greater, 4.0% by mass or greater, 4.5% by mass or greater, 5.0% by mass or greater, 5.5% by mass or greater, 6.0% by mass or greater, 6.5% by mass or greater, 7.0% by mass or greater, 7.5% by mass or greater, 8.0% by mass or greater, 8.5% by mass or greater, 9.0% by mass or greater, 9.5% by mass or greater, or 10% by mass or greater, and may be 20% by mass or less, 15% by mass or less, 10% by mass or less, 8.0% by mass or less, 5.0% by mass or less, or 2.0% by mass or less, relative to the entire composition.

### <Component (C): oil>

The composition of the present invention comprises one or more oils. In the present invention, the oil preferably comprises an oil capable of at least partially dissolving Compound I described above.

In the composition of the present invention, the content of the oil is not particularly limited and, for example, may be 5.0% by mass or greater, 10% by mass or greater, 15% by mass or greater, 20% by mass or greater, 25% by mass or greater, 30% by mass or greater, 35% by mass or greater, 40% by mass or greater, or 50% by mass or greater, and may be 99.9% by mass or less, 90% by mass or less, 80% by mass or less, 70% by mass or less, 60% by mass or less, 55% by mass or less, 50% by mass or less, 45% by mass or less, 40% by mass or less, 35% by mass or less, or 30% by mass or less. The content of the oil may be appropriately adjusted in accordance with the desired dosage form (for example, water-in-oil cosmetic composition or oil-in-water cosmetic composition) of the composition of the present invention.

In the present invention, the oil, for example, may comprise one or more of a polar oil, a silicone oil, and a hydrocarbon oil. From the viewpoint of dissolving Compound I described above, the oil preferably comprises a polar oil.

### (Polar oil)

In the present invention, "polar oil" refers to a highly polar oil, among oils that can be used in cosmetics, other than a silicone oil, and refers to one having, for example, an IOB value of 0.10 or greater, 0.11 or greater, 0.12 or greater, or 0.13 or greater. The IOB value of the polar oil according to the present invention may be 1.50 or less, 1.00 or less, 0.50 or less, 0.45 or less, or 0.40 or less. IOB value is an abbreviation for inorganic/organic balance, is a value that represents a ratio of inorganic value to organic value, and acts as an indicator showing the degree of polarity of an organic compound. The IOB value, specifically, is represented as IOB value = inorganic value / organic value. With respect to each of "inorganic value" and "organic value", the "inorganic value" and "organic value" are set depending on various atoms or functional groups, such that, for example, the "organic value" for one carbon atom in a molecule is 20, and the "inorganic value" for one hydroxyl group is 100, and by summing the "inorganic values" and "organic values" of all atoms and functional groups in an organic compound, the IOB value of the organic compound can be calculated (refer to, for example, Yoshio Koda, "Organic Conceptual Diagrams - Fundamentals and Applications -", pp. 11 to 17, Sankyo Publishing Co., Ltd., published in 1984).

In the present invention, the polar oil, for example, may be at least one selected from the group consisting of ester oils, ether oils, higher alcohols, and fatty acids, having an IOB value of 0.10 or greater.

More specifically, the polar oil, for example, may be at least one selected from the group consisting of bis-ethoxydiglycol cyclohexane-1,4-dicarboxylate (IOB value = 1.03), phenoxyethyl caprylate (IOB value = 0.29), PPG-30-buteth-30 (IOB value = 0.94), diisopropyl sebacate (IOB value = 0.4), neopentyl glycol diheptanoate (IOB value = 0.33), diethylhexyl succinate (IOB value = 0.32), isononyl isononanoate (IOB value = 0.2), isopropyl myristate (IOB value = 0.18), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), and glyceryl tri(caprylate/caprate) (IOB value = 0.33), but are not limited thereto.

In the present invention, the amount of polar oil contained in the oil is not particularly limited and, for example, may be 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less, relative to 100 parts by mass in total of oil.

When the oil in the composition of the present invention comprises a polar oil, the polar oil and Compound I described above may be blended in the following compositional ratio. More specifically, Compound I may be 0.01 parts by mass or more, 0.05 parts by mass or more, 0.1 parts by mass or more, 0.5 parts by mass or more, 1.0 parts by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, 9.5 parts by mass or more, 10 parts by mass or more, 15 parts by mass or more, 20 parts by mass or more, 25 parts by mass or more, or 30 parts by mass or more, and may be 50 parts by mass or less, 45 parts by mass or less, 40 parts by mass or less, 35 parts by mass or less, 30 parts by mass or less, 25 parts by mass or less, 20 parts by mass or less, 15 parts by mass or less, or 10 parts by mass or less, relative to 100 parts by mass in total of the polar oil and Compound I.

In the present invention, when using an ultraviolet absorber as the polar oil, particularly when using a UVB absorber, the amount of UVB absorber in the oil may be 50 parts by mass or less, 40 parts by mass or less, 30 parts by mass or less, 20 parts by mass or less, 10 parts by mass or less, 5.0 parts by mass or less, 1.0 parts by mass or less, 0.5 parts by mass or less, 0.1 parts by mass or less, 0.01 parts by mass or less, or 0.001 parts by mass or less, relative to 100 parts by mass of oil.

### (Silicone oil)

In the present invention, silicone oil refers to an oil having a main backbone formed by siloxane bonds, among oils that can be used in cosmetics.

In the present invention, the silicone oil may be a volatile silicone oil, or may be a non-volatile silicone oil.

In the present invention, as a guideline for "volatile", a boiling point under 1 atmosphere (101.325 kPa) can be used. This boiling point, for example, may be 250°C or lower, 240°C or lower, or 230°C or lower, or may be 80°C or higher, 100°C or higher, 120°C or higher, 150°C or higher, or 160°C or higher. In the present disclosure, "non-volatile" refers to a substance that exhibits a volatile content of 5% or less when a sample is placed in a sufficiently large flat-bottomed dish and left to stand at 105°C for 3 hours.

In the present invention, the silicone oil may be a non-cyclic silicone oil (i.e., a linear silicone oil), or may be a cyclic silicone oil.

Specific examples of the silicone oil include non-cyclic silicone oils such as polydimethylsiloxane (dimethicone), trisiloxane, caprylyl methicone, methylphenyl polysiloxane, and methylhydrogenpolysiloxane; cyclic silicone oils such as octamethylcyclotetrasiloxane, decamethylcyclopentasiloxane, and dodecamethylcyclohexasiloxane; and combinations of two or more thereof, but are not limited thereto. From the viewpoint of feel of use, as the silicone oil, a non-cyclic silicone oil is particularly preferable.

In the present invention, the amount of silicone oil contained in the oil is not particularly limited and, for example, may be 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less, relative to 100 parts by mass in total of oil.

### (Hydrocarbon oil)

In the present invention, hydrocarbon oil refers to a hydrocarbon oil other than the polar oil described above.

The hydrocarbon oil may be a volatile hydrocarbon oil, or may be a non-volatile hydrocarbon oil.

Specific examples of the hydrocarbon oil include decane, dodecane, isododecane, isohexadecane, liquid paraffin, squalane, squalene, paraffin, hydrogenated polydecene, and mixtures of two or more thereof, but are not limited thereto.

### <Additional components>

The composition of the present invention, in addition to the components described above, may further optionally comprise additional components as long as the effect of the present invention is not impaired. In the following, the additional components will be described by way of example. However, the present invention is not limited to these additional components.

### (Component (D): UVA absorber)

The composition of the present invention preferably further comprises a UVA absorber. According to intensive research by the present inventors, by using a UVA absorber in combination with Compound I described above, it was found that the effect of the present invention can be further improved.

In the present invention, the UVA absorber is not particularly limited, and specific examples can include methylene bis-benzotriazolyl tetramethylbutylphenol, t-butyl methoxydibenzoylmethane, diethylamino hydroxybenzoyl hexyl benzoate, and bis-ethylhexyloxyphenol methoxyphenyl triazine. The UVA absorber preferably comprises one or more selected from the group consisting of t-butyl methoxydibenzoylmethane, diethylamino hydroxybenzoyl hexyl benzoate, and bis-ethylhexyloxyphenol methoxyphenyl triazine, and more preferably comprises at least bis-ethylhexyloxyphenol methoxyphenyl triazine or comprises bis-ethylhexyloxyphenol methoxyphenyl triazine and diethylamino hydroxybenzoyl hexyl benzoate.

In the composition of the present invention, the content of the UVA absorber, if comprising any, is not particularly limited and, for example, 0.01% by mass or greater, 0.05% by mass or greater, 0.1% by mass or greater, 0.5% by mass or greater, or 1.0% by mass or greater, and may be 20% by mass or less, 15% by mass or less, 10% by mass or less, 5.0% by mass or less, or 1.0% by mass or less, relative to the entire composition.

In the composition of the present invention, if comprising a UVA absorber, the ratio of the content of UVA absorber to the content of Compound I (UVA absorber : Compound I) is not particularly limited and, for example, may be 1:100 to 1:1, 1:50 to 1:1, 1:10 to 1:2, or 1:5 to 1:2.

### (Water)

The composition of the present invention may further comprise water. The water is not particularly limited and, for example, may be water used in cosmetics and quasi-drugs. For example, ion-exchanged water, distilled water, ultrapure water, or tap water can be used.

The content of water, if comprising any, is not particularly limited, and may be appropriately adjusted in consideration of the balance with the content thereof in oil, in accordance with the desired dosage form (for example, water-in-oil cosmetic composition or oil-in-water cosmetic composition) of the composition of the present invention.

### (Surfactant)

In the present invention, the surfactant may be any of those that function as an emulsifier, those that function as a dispersant, and those that serve the functions of an emulsifier and a dispersant. The surfactant may be any of an anionic surfactant, a cationic surfactant, an amphoteric surfactant, and a nonionic surfactant.

In the composition of the present invention, the content of the surfactant, if comprising any, is not particularly limited and, for example, may be 0.01% by mass or greater, 0.05% by mass or greater, 0.1% by mass or greater, 0.5% by mass or greater, 1.0% by mass or greater, 1.5% by mass or greater, or 2.0% by mass or greater, and may be 30% by mass or less, 20% by mass or less, 15% by mass or less, 10% by mass or less, 5.0% by mass or less, 4.0% by mass or less, 3.0% by mass or less, 2.0% by mass or less, or 1.0% by mass or less, relative to the entire composition.

### (Alcohol component)

The composition of the present invention can further comprise an alcohol component. In the present invention, the definition of "alcohol component" includes both lower alcohols and polyhydric alcohols. These alcohol components can be mixed with water, can also be referred to as water-soluble components, and can constitute an aqueous phase together with water. A portion of the alcohol component can have a moisturizing function, i.e., can be used as a moisturizer.

In the present invention, lower alcohol refers to an alcohol having 5 or fewer carbon atoms, and is exemplified by methanol, ethanol, propanol, isopropanol, butanol, isobutanol, and t-butanol.

Examples of polyhydric alcohols include dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritol such as 1,2,6-hexanetriol); pentahydric alcohols (for example, xylitol (also classified as a sugar alcohol)); and hexahydric alcohols (for example, sorbitol and mannitol (both also classified as sugar alcohols)), but are not limited thereto.

In the composition of the present invention, the content of the alcohol component, if comprising any, is not particularly limited and, for example, may be 0.5% by mass or greater, 1.0% by mass or greater, 5.0% by mass or greater, 10% by mass or greater, 15% by mass or greater, or 20% by mass or greater, and may be 40% by mass or less, 30% by mass or less, or 25% by mass or less, relative to the entire composition.

### (Further optional additive component)

The composition of the present invention, in addition to the components above, can be appropriately blended, as needed, with other optional additive components commonly used in skin external preparations such as cosmetics and pharmaceuticals, in ranges not impairing the purpose and effect of the present invention, for example, thickeners, chelating agents, pH adjusters, antioxidants, powder components (for example, hydrophobically treated silica), and fragrances, but are not limited to these examples.

Examples of thickeners include plant-based polymers, vinyl-based polymers, acrylic polymers, amino acid-based gelling agents, sugar fatty acid esters, waxes, fatty acids that are solid at room temperature, fatty acid esters that are solid at room temperature, and organically modified clay minerals, but are not limited thereto.

Examples of chelating agents include sodium edetate and sodium metaphosphate, but are not limited thereto.

Examples of pH adjusters include buffering agents such as lactic acid-sodium lactate and citric acid-sodium citrate; and amino acids (for example, glycine), but are not limited thereto.

Examples of antioxidants include dibutylhydroxytoluene (BHT) and butylhydroxyanisole (BHA), but are not limited thereto.

### <Dosage form and application of composition of present invention>

The dosage form of the composition of the present invention is not particularly limited and, for example, may be an oil-in-water emulsion cosmetic composition, a water-in-oil emulsion cosmetic composition, or an oily cosmetic composition based on an oil agent.

The composition of the present invention can be suitably used as a cosmetic or a raw material thereof. Thus, the present invention can be used as a sun-blocking cosmetic composition such as a cosmetic primer or a sunscreen. The product form of these cosmetics is not particularly limited, and may be in cream form, emulsion form, mist form, oily solid form, or gel form.

The manufacturing method for the composition of the present invention is not particularly limited, and can be carried out by a conventional method in accordance with the desired dosage form (for example, water-in-oil cosmetic composition or oil-in-water cosmetic composition).

### EXAMPLES

The present invention will be further described in detail with reference to the Examples below. However, the present invention is not limited thereto. Unless specified otherwise, the numerical values in tables showing formulations represent blending amounts of each component, and are expressed as % by mass.

### <<Examples 1 to 4 and Comparative Examples 1 to 5>>

Based on the formulations shown in Table 1, as water-in-oil cosmetic compositions, the compositions of Examples 1 to 4, Comparative Examples 1 to 5, and Control Compositions 1 and 2 were prepared.

Cumulative fluorescence value for each of the obtained compositions was measured as follows. For Examples 1 to 3 and Comparative Examples 1 to 4, ratios (%) of cumulative fluorescence value relative to Control Composition 1 were calculated, and for Example 4 and Comparative Example 5, ratios of cumulative fluorescence value relative to Control Composition 2 were calculated. Results are shown in Table 1.

### (Measurement of cumulative fluorescence value)

Each composition was applied to an S plate (refer to Japanese Registered Patent Publication No. 4453995) at 2 mg/cm² and dried to prepare a coating of the composition. The obtained coating was irradiated with ultraviolet rays having a wavelength of 365 nm, and the cumulative fluorescence value in the wavelength region of 400 to 600 nm was measured using an RF-5300PC spectrofluorometer (Shimadzu Corporation).

The ultraviolet absorption wavelength of each of the obtained compositions was measured separately as follows to confirm the ultraviolet absorbance at 365 nm. The obtained comparison results are shown in Table 1.

### (Measurement of ultraviolet absorbance at 365 nm)

Each composition was applied to an S plate and dried to prepare a coating of the composition. Using a Model U-3500 spectrophotometer manufactured by Hitachi, Ltd., the ultraviolet absorbance at 365 nm of each composition was measured.

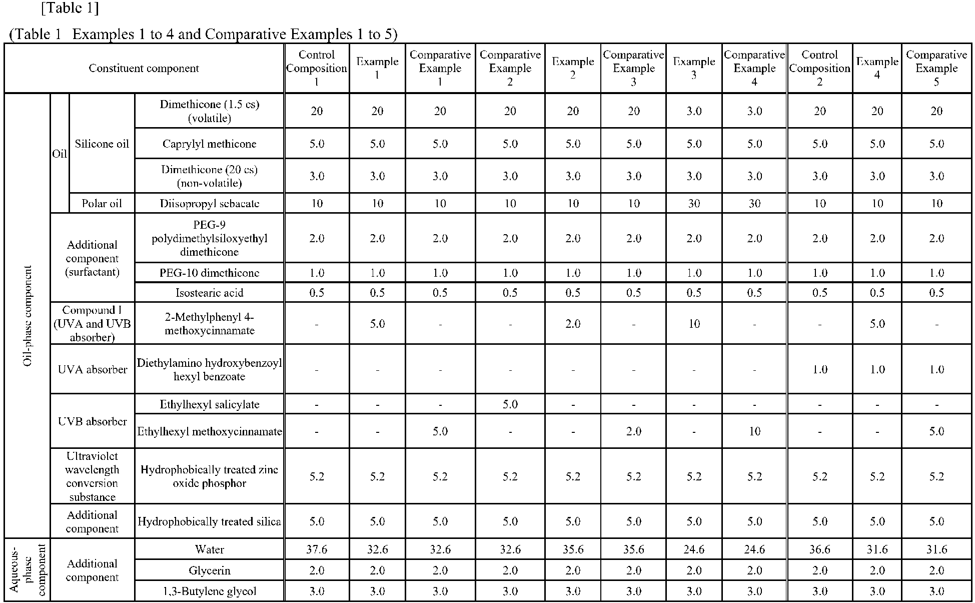

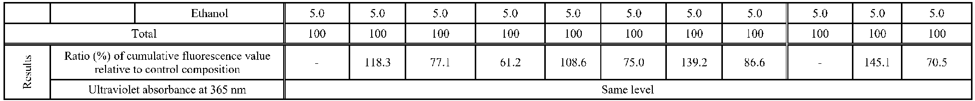

As is clear from the results in Table 1, the compositions of Examples 1 to 3 comprising Compound I as a UVA and UVB absorber all had high ratios of cumulative fluorescence value relative to Control Composition 1, exceeding 100%. In contrast, Comparative Examples 1 to 4, which did not comprise Compound I and comprised other UVB absorbers, all had low ratios of cumulative fluorescence value relative to Control Composition 1. The composition of Example 4 comprising Compound I and a UVA absorber had the highest ratio of cumulative fluorescence value relative to Control Composition 2, exceeding 145%. In contrast, Comparative Example 5 comprising a UVB absorber other than Compound I had a low ratio of cumulative fluorescence value relative to Control Composition 2.

From the above results, it was suggested that when an ultraviolet absorber (for example, a UVB absorber) other than Compound I is used in combination with a zinc oxide phosphor, the wavelength conversion efficiency of the zinc oxide phosphor deteriorates, compared to when such an ultraviolet absorber is not used in combination. It was suggested that by blending Compound I as an ultraviolet absorber, wavelength conversion efficiency of a zinc oxide phosphor is significantly improved.

It was further suggested that by using Compound I and a UVA absorber in combination, wavelength conversion efficiency of a zinc oxide phosphor is even further improved. The results of ultraviolet absorbance at 365 nm were substantially the same for all of the compositions.

### <<Example 5 and Comparative Example 6>>

Based on the formulations shown in Table 2, as the oil-in-water cosmetic compositions, the compositions of Example 5 and Comparative Example 6, as well as Control Composition 3, were prepared.

The cumulative fluorescence value for each of the obtained compositions was measured in the same manner as above, and a ratio of cumulative fluorescence value relative to Control Composition 3 was calculated. The ultraviolet absorption wavelength was measured in the same manner as above to confirm the ultraviolet absorbance at 365 nm. These results are shown in Table 2.

**[Table 2]**

| (Table 2 Example 5 and Comparative Example 6) | | | | | |
|---|---|---|---|---|---|
| Constituent component | | | Control Composition 3 | Example 5 | Comparative Example 6 |
| Water | | Water | 51.68 | 46.68 | 46.68 |
| Ultraviolet wavelength conversion substance | | Zinc oxide phosphor | 5.2 | 5.2 | 5.2 |
| Oil | Polar oil | Diisopropyl sebacate | 10 | 10 | 10 |
| | Hydrocarbon oil | Hydrogenated polydecene | 6.0 | 6.0 | 6.0 |
| UVB absorber | | Ethylhexyl methoxycinnamate | - | - | 5.0 |
| Compound I (UVA and UVB absorber) | | 2-Methylphenyl 4-methoxycinnamate | - | 5.0 | - |
| Additional components | | | Appropriate amount | Appropriate amount | Appropriate amount |
| Total | | | 100 | 100 | 100 |
| Results | | Ratio (%) of cumulative fluorescence value relative to control composition | - | 112.3 | 93.5 |
| | | Ultraviolet absorbance at 365 nm | Same level | | |

Details of "additional components" in Table 2 are as follows:
· Citric acid (foodstuff) appropriate amount
· Sodium citrate appropriate amount
· EDTA-2Na·2H₂O appropriate amount
· Ethanol 5.0% by mass
· Phenoxyethanol 0.5% by mass
· Silica 3.0% by mass
· BG 6.0% by mass
· PEG-100 hydrogenated castor oil 1.5% by mass
· Glycerin 3.0% by mass
· Hydroxypropyl methylcellulose stearoxy ether 0.1% by mass
· Succinoglycan 0.15% by mass
· (Dimethylacrylamide/sodium acryloyldimethyltaurate) crosspolymer 0.25% by mass
· Alkyl benzoate (C12-15) 3.0% by mass
· Dextrin (palmitate/ethylhexanoate) 0.5% by mass
· Triethylhexanoin 3.0% by mass
· PPG-17 1.0% by mass

As is clear from the results in Table 2, the composition of Example 5 comprising Compound I as a UVA and UVB absorber had a ratio of cumulative fluorescence value relative to Control Composition 3 that exceeded 110%. In contrast, the composition of Comparative Example 6 comprising a UVB absorber other than Compound I had a low ratio of cumulative fluorescence value relative to Control Composition 3.

From these results, it was suggested that when an ultraviolet absorber other than Compound I is used in combination with a zinc oxide phosphor, the wavelength conversion efficiency of the zinc oxide phosphor deteriorates, compared to when such an ultraviolet absorber is not used in combination. It was suggested that by blending Compound I, wavelength conversion efficiency of a zinc oxide phosphor can be significantly improved.

Summarizing the results in Table 1 and Table 2 above, it was found that the compositions of Examples 1 to 5, which use Compound I in combination with a zinc oxide phosphor as an ultraviolet wavelength conversion substance, all have an ultraviolet protection effect while also being capable of improving the wavelength conversion efficiency of the ultraviolet wavelength conversion substance.

## Claims

1. A cosmetic composition, comprising
(A) an ultraviolet wavelength conversion substance;
(B) Compound I having a structure of formula (I) below: 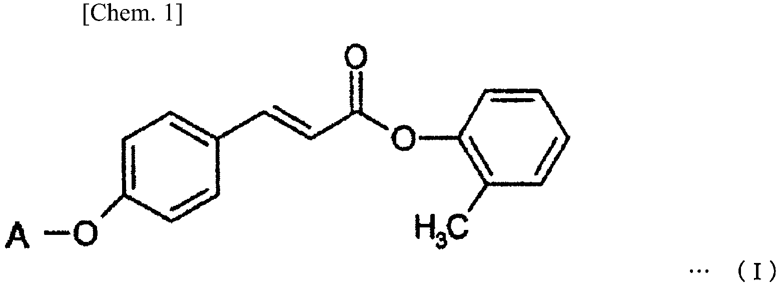 wherein -OA represents an alkoxy group; and
(C) an oil.

2. The composition according to claim 1, wherein the component (A) is a zinc oxide phosphor.

3. The composition according to claim 1, wherein a content of the component (B) is 0.5 to 20% by mass.

4. The composition according to claim 1, wherein the component (B) is 2-methylphenyl 4-methoxycinnamate.

5. The composition according to claim 1, further comprising (D) a UVA absorber.

6. The composition according to claim 1, wherein the component (C) comprises a polar oil.

7. The composition according to any one of claims 1 to 6, which is a water-in-oil cosmetic composition.

8. The composition according to any one of claims 1 to 6, which is an oil-in-water cosmetic composition.
